# EUROPEAN PATENT SPECIFICATION

(11) **EP 3 111 246 B1**
(45) Date of publication and mention of the grant of the patent: **25.06.2025**
(21) Application number: 15755714.1
(22) Date of filing: 26.02.2015
(51) Int. Cl.: G01S 5/02, G01S 13/76, G08B 25/00, H04L 9/00, H04N 5/76, H04W 8/26, H04W 16/26, H04W 92/02, G01S 13/87, G07C 9/27, G08B 7/06, H04L 101/672, H04L 43/0805, H04L 61/106, H04L 67/104, H04L 67/12, H04L 67/00, G08B 13/196, G08B 29/18, H04W 4/00, H04W 84/18, H04W 88/04

(54) **REAL-TIME LOCATION SYSTEM IN WIRELESS SENSOR NETWORK**
ECHTZEIT-POSITIONSFINDUNGSSYSTEM IN EINEM DRAHTLOSEN SENSORNETZWERK
SYSTÈME DE LOCALISATION EN TEMPS RÉEL DANS UN RÉSEAU DE CAPTEURS SANS FIL

(30) Priority: 28.02.2014 US 201461946054 P; 02.04.2014 US 201461973962 P; 20.08.2014 US 201414464010
(43) Date of publication of application: 04.01.2017
(73) Proprietor: Tyco Fire & Security GmbH, 8212 Neuhausen am Rheinfall (CH)
(72) Inventor: HALL, Stewart E., Wellington, FL 33414 (US); PATTERSON, Hap, Boca Raton, FL 33431 (US); BREWIN, Robert F., San Diego, CA 92127 (US)
(74) Representative: Meissner Bolte Nürnberg
(86) International application number: PCT/US2015/017702
(87) International publication number: WO 2015/130910

(56) References cited:
- WO-A1-01/06401
- WO-A1-2013/159217
- US-A1- 2004 027 243
- US-A1- 2005 052 281
- US-A1- 2007 239 350
- US-A1- 2008 068 267
- US-B1- 8 400 268

## Description

This description relates to operation of security systems in particular intrusion systems.

### Background

This description relates to operation of security systems in particular physical intrusion and alarm systems installed on commercial or residential premises.

It is common for businesses and homeowners to have a security system for detecting alarm conditions at their premises and signaling the conditions to a monitoring station or to authorized users of the security system. Sensors types typically include motion detectors, cameras, and proximity sensors (used to determine whether a door or window has been opened). One particular type of senor is a badge or tag reader to track movement of an credentialed individual within a premises, such as in a major commercial or industrial facility.

Real-time location systems (RTLS) use an active system to determine the current location of a moving tag within an environment. Different systems use different technologies to determine the location, but such systems typically use either distance measurement or angle measurement (or a combination of the two) between two or more elements in the system with known locations (e.g., locating elements) and the element in the system that is being located (e.g., a tag).

US 2005/0052281 AI discloses a location tracking system for tracking the location of items within a controlled area which comprises a plurality of RFID tags located according to the required accuracy of the location determinations. Vehicles configured to transport items being tracked include two RFID interrogators configured to acquire RFID information from the plurality of RFID tags and to transmit the RFID tag information to a location authority. The separation of the two RFID interrogators is set based on the spacing of the plurality of RFID tags such that the required accuracy results.

US 8,400,268 B1 discloses a system comprising a plurality of reference tags affixed to reference locations in a coverage area and configured to form a wireless mesh network. The system also includes a call tag configured to communicate with the plurality of reference tags and to collect data regarding the communication with the plurality of reference tags. The call tag is configured to send a first signal in response to an event. A positioning system is coupled to the wireless mesh network and configured to receive the first signal and the data collected by the call tag. The positioning system is further configured to process the data collected by the call tag to determine the call tag location with respect to the plurality of reference tags.

US 2004/0027243 A1 discloses a system and method for acquisition management of subject position information that utilizes RFID to store position information in position tags. Tag programmers receive position information from external positioning systems, such as GPS, from manual inputs, such as keypads, or other tag programmers. The tag programmers program each position tag with the received position information. Both the tag programmers and the position tags can be portable or fixed.

US 2008/0056261 A1 discloses a gateway which enables communication between a personal area network and an Internet Protocol network. The gateway includes a first interface device for connecting to the personal area network, a second interface device for connecting to the IP network, and a gateway controller. In one embodiment, the gateway controller allocates ports on an IP interface to one or more clients in said personal area network, stores a routing table in memory for relating said clients in said personal area network with their corresponding ports, and transfers messages between said personal area network clients and said IP network based on entries in said routing table. The gateway may function in conjunction with a gateway proxy.

EP 1885039 A2 discloses an emergency system for providing an emergency function in which the system is required to provide an autonomous and self-diagnostic capability so the system can be tested when not in operation in an emergency situation. A plurality of emergency devices are provided which have transmitters and receivers for producing a mesh network. Each device has a processor for establishing a preferred wireless communication link of the mesh network to another device for relaying signals and messages. The devices are arranged in subnets and the subnets are also grouped into groups of devices which may cross various subnets so that only one group of devices can be tested at a particular time.

### SUMMARY

Several problems limit the efficacy of real-time location systems (RTLS). A traditional RTLS system depends on having accurate position information for each of the locating elements. These locating elements either need to be fixed at an *a priori* known location or if they are portable elements, the elements location needs top be accurately determined at setup time. GPS is often used to determine the location of portable locating elements. However that is only possible in locations where GPS is functional and accurate (i.e. outdoors without obstructions).

The difficulty of setting up and determining the position the locating elements requires a person who is highly trained in the use of the system and takes a significant amount of time to deploy. The distance between the Locating Elements and the Tag is limited by a number of factors depending on the technology that is used. For line of sight systems such as laser based ranging systems, there can be no obstructions. Even for systems that can penetrate obstructions such as those using Radiating Electromagnetic energy (RF), obstructions decrease the range of these applications. The accuracy of the tag location is often compromised by a phenomenon called multipath which causes reflected signals to be picked up by the receiver which interfere with the signal enough to cause a decrease in the accuracy of the calculated location or make the location completely inaccurate. The location information that is received by the system is typically provided in geographic coordinates. To use this location information, a system translates these coordinates into a location on a map or floor plan of a building such as "on the second floor near the north stairwell of the town hall building."

According to an aspect of the present invention, a real-time location system according to claim 1 is provided.

According to an additional aspect of the present invention, a method of real-time location tracking according to claim 10 is provided.

The disclosed real-time location system resolves or mitigates these issues by changing the way the system is deployed and decoupling the setup process from the tag location finding process.

The details of one or more embodiments of the invention are set forth in the accompanying drawings and the description below. Other features, objects, and advantages of the invention is apparent from the description and drawings, and from the claims.

### DESCRIPTION OF DRAWINGS

FIG. 1 is a schematic diagram of an exemplary networked security system.
FIG. 2 is a block diagram of a real-time location system.
FIGS. 3-7 are block diagrams of components of the real-time location system of FIG. 2.
FIG. 8 is an exemplary layout plan useful in understanding setup.
FIGS. 9-12 are diagrams useful in understanding setup and operation of the RTL system of FIG. 2.
FIG. 13 is a block diagram of an example sensor network for physical premises security applications.

### DETAILED DESCRIPTION

Described herein are examples of network features that may be used in various contexts including, but not limited to, security/intrusion and alarm systems. Example security systems may include an intrusion detection panel that is electrically or wirelessly connected to a variety of sensors. Those sensors types may include motion detectors, cameras, and proximity sensors (used, e.g., to determine whether a door or window has been opened). Typically, such systems receive a relatively simple signal (electrically open or closed) from one or more of these sensors to indicate that a particular condition being monitored has changed or become unsecure.

For example, typical intrusion systems can be set-up to monitor entry doors in a building. When a door is secured, a proximity sensor senses a magnetic contact and produces an electrically closed circuit. When the door is opened, the proximity sensor opens the circuit, and sends a signal to the panel indicating that an alarm condition has occurred (e.g., an opened entry door).

Data collection systems are becoming more common in some applications, such as home safety monitoring. Data collection systems employ wireless sensor networks and wireless devices, and may include remote server-based monitoring and report generation. As described in more detail below, wireless sensor networks generally use a combination of wired and wireless links between computing devices, with wireless links usually used for the lowest level connections (e.g., end-node device to hub/gateway). In an example network, the edge (wirelessly-connected) tier of the network is comprised of resource-constrained devices with specific functions. These devices may have a small-to-moderate amount of processing power and memory, and may be battery powered, thus requiring that they conserve energy by spending much of their time in sleep mode. A typical model is one where the edge devices generally form a single wireless network in which each end-node communicates directly with its parent node in a hub-and-spoke-style architecture. The parent node may be, e.g., an access point on a gateway or a sub-coordinator which is, in turn, connected to the access point or another sub-coordinator.

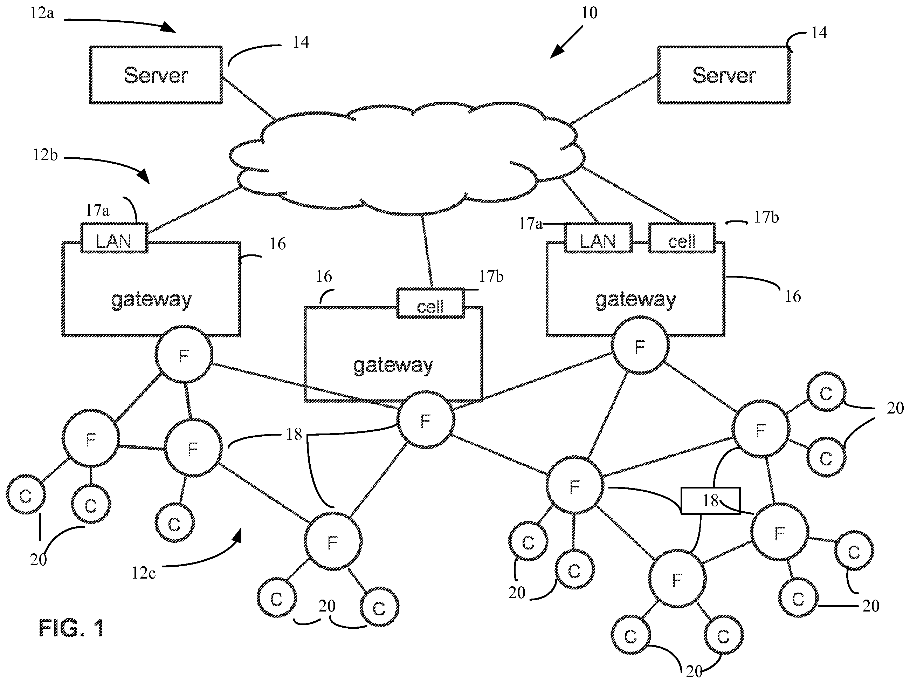

Referring now to FIG. 1, an exemplary (global) distributed network topology for a Wireless Sensor Network (WSN) is shown. In FIG. 1 the distributed network 10 is logically divided into a set of tiers or hierarchical levels 12a-12c.

The global distributed network topology for the sensor network includes distributed rule engines denoted by the circle element "R" at individual nodes or collections of nodes. In an upper tier or hierarchical level 12a of the network are disposed servers and/or virtual servers 14 running a "cloud computing" paradigm that are networked together using well-established networking technology such as Internet protocols or which can be private networks that use none or part of the Internet.

Applications that run on those servers 14 communicate using various protocols such as for Web Internet networks XML/SOAP, RESTful web service, and other application layer technologies such as HTTP and ATOM. The distributed network 10 has direct links between devices (nodes) as shown and discussed below.

The distributed network 10 includes a second logically divided tier or hierarchical level 12b, referred to here as a middle tier that involves gateways 16 located at central, convenient places inside individual buildings and structures. These gateways 16 communicate with servers 14 in the upper tier whether the servers are stand-alone dedicated servers and/or cloud based servers running cloud applications using web programming techniques. The middle tier gateways 16 are also shown with both local area network 17a (e.g., Ethernet or 802.11) and cellular network interfaces 17b.

The distributed network topology also includes a lower tier (edge layer) 12c set of devices that involve fully-functional sensor nodes 18 (e.g., sensor nodes that include wireless devices, e.g., transceivers or at least transmitters, which in FIG. 1 are marked in with an "F") as well as constrained wireless sensor nodes or sensor end-nodes (marked in the FIG. 1 with "C"). In some examples wired sensors (not shown) can be included in aspects of the distributed network 10.

### Real-time location system

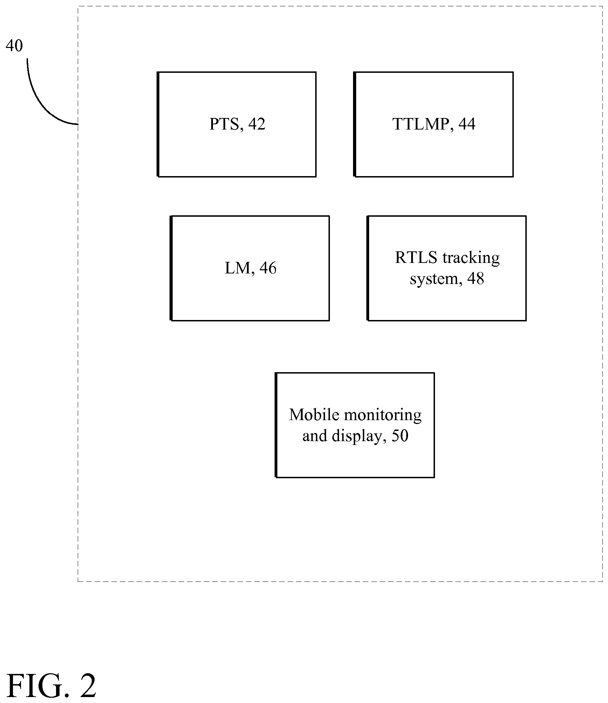

Referring now to FIG. 2, a real-time location system 40 using stationary, wireless sensor nodes and a portable transceiver is described. The system 40 includes a positioning transceiver system (PTS) 42, a tag transceiver with location marker programmer (TTLMP) 44, a location marker (LM) 46, a mobile tracking system 48 and a mobile monitoring and display system 50.

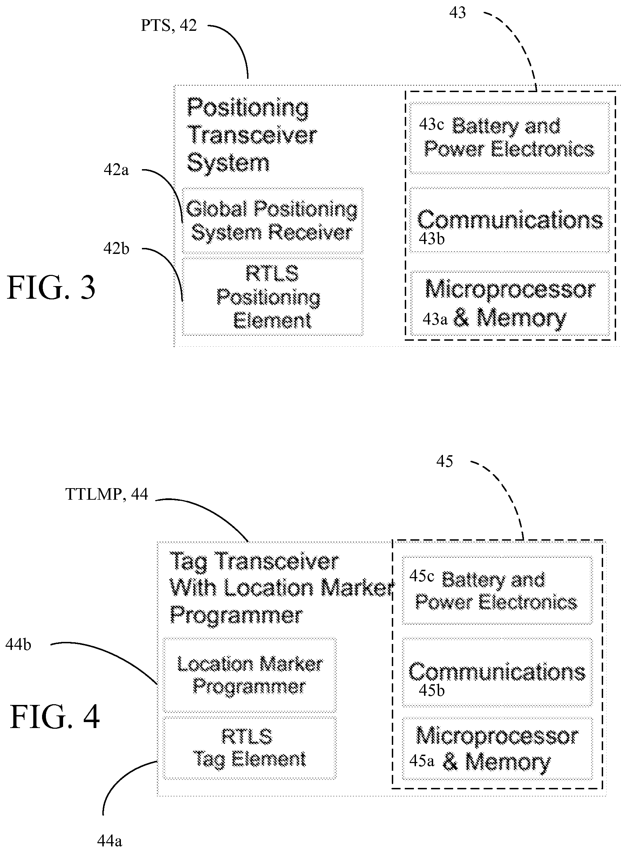

Referring to FIG. 3, the positioning transceiver system 42 includes a subsystem 42a that determines the position of the positioning transceiver system 42 relative to a global coordinate system such as latitude and longitude. An example of such a system would be a GPS receiver. The PTS 42 also includes a traditional positioning element 42b that is used to determine the distance or angle to the traditional tag. The system 42 also includes circuitry 43 including a microprocessor and memory subsystem 43a that is used to control the elements of the positioning transceiver system 42, (as well as a Battery and Power Electronics subsystem 43c that supplies power to the system during operation and a communications subsystem 43b that provides data and time synchronization between the various other Positioning Transceiver Systems (not shown), as well as communication of data to the Tagging Transceiver with Location Marker Programmer 44.

Referring to FIG. 4, the tag transceiver with location marker programmer 44 includes an Tag Element 44a that together with the Positioning Elements 42b (FIG. 3) are used to determine the location of the tag transceiver with location marker programmer 44 relative to the global position coordinates, a Location Marker Programmer 44b that is used to program a location marker with the coordinates and user-friendly location information. The tag transceiver with location marker programmer 44 also includes circuitry 45 including microprocessor and memory subsystem 45a (as well as a Battery and Power Electronics subsystem 45c) and a communications subsystem 45b that is used to control the elements of the Tag Transceiver 44a and Location Marker Programmer 44b, supply power to the Tag Transceiver 44a and Location Marker Programmer 44b during operation with the communications subsystem providing data and time synchronization between various Positioning Transceiver Systems.

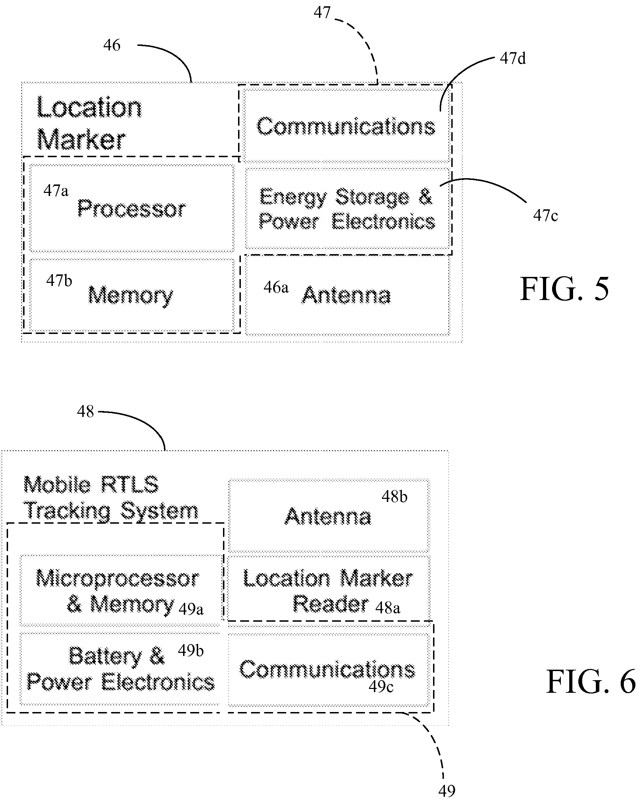

Referring to FIG. 5, the Location Marker 46 is shown. The location marker 46 is a low cost element that is programmed with geo coordinates of the location that the location marker 46 will be affixed to and/or a user-friendly location (e.g., a user supplied name) by the tag transceiver with location marker programmer 44 (FIG. 4). The location marker 46 includes circuitry 47 including a processor 47a that controls functionality of the location marker 46 and memory 47b that can be programmed with the geo coordinates and / or the friendly location information by the Tag Transceiver with Location Marker Programmer 46 and a communications block 47d that communicates with the Tag Transceiver with Location Marker Programmer 46.

Also included in the location marker 46 is an antenna 46a for the communication block 47d. The antenna 46a can be configured to provide different sensitivity patterns to allow for directional reading. The location marker 46 also includes an energy Storage and Power Electronics module 47c that provides power to the system during operation. The Location marker 46 may be either powered with an internal battery, an external power source or powered by harvesting power from the Tag Transceiver with Location Marker Programmer 46 during programming and from the mobile monitoring and display system 50 (FIG. 2) during the tracking phase, as discussed below.

Referring to FIG. 6, a mobile tracking system 48 is carried by a user and is used to read location information from nearby location markers 46 that are placed around a site. The mobile tracking system 48 communicates the geo coordinates and / or the user-friendly location information to the mobile monitoring and display system 50 during the tracking phase of operation. The mobile tracking system 48 includes a location marker reader 48a that reads information from location markers 46 during the tracking phase of operation, an antenna 48b that facilitates communication with the Location markers 46 and circuitry 49 including a microprocessor and memory 49a that controls the operation of the individual components of the mobile tracking system 48. Also included in the circuitry 49 are a battery and power electronic block 49b that provides power to the system 48 and a communications block 49c that provides a communication channel between the mobile tracking system 48 and the mobile monitoring and display system 50.

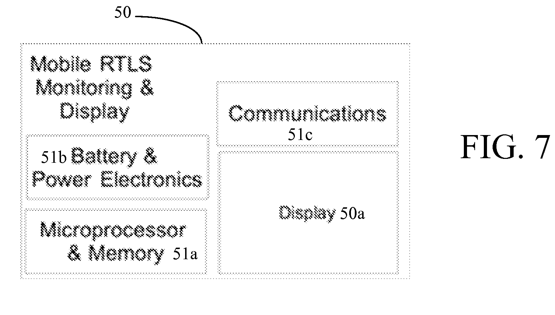

Referring to FIG. 7, a mobile monitoring and display system 50 displays the status and geo coordinates and / or user friendly location of one or more mobile tracking systems on a display which may also display maps or floor plans of a site or building. The mobile monitoring and display system 50 includes a display 50a that communicates visual and / or audible information about the geo coordinates and / or the user-friendly location information of the mobile tracking systems 48 that are associated with the mobile monitoring and display system 50. The mobile monitoring and display system 50 also includes a microprocessor and memory 51a that controls the functionality of the mobile monitoring and display system 50 and battery and power electronics 51b that provides power to operate the mobile monitoring and display system 50 and a communications block 51c that provides a communication channel to one or more mobile tracking systems that are associated with this mobile monitoring and display system 50.

### Description of the Svstem Operation

### The Deployment and Setup Phase of Operation

Setting up the system 40 involves setting up several Positioning Transceiver System elements 42 around a site or building that is to be so equipped. Each of these elements is placed in a known geographic location or in a location where a geographic position receiver system such as GPS would have ability to accurately determine their geo coordinates. The Positioning Transceiver Elements are placed in locations that would be within range of the areas of the site or building to be surveyed and equipped with location markers 46.

Once the positioning transceiver system 42 are placed throughout the site, a user begins a survey by taking a tag transceiver with location marker programmer 44 to various locations around the site that are to be tagged with location markers 46. Typically the location markers 46 will be placed in locations such as hallways, doorways, elevators, stairways and other choke points where traffic will move during the tracking phase of operation. When a location is found to place a location marker, the tag transceiver with location marker programmer 44 determines the geo coordinates of that location and/or the user enters a user-friendly name (such as "Northwest Stairway, 2^{nd} Floor") into the tag transceiver with location marker programmer 44. The location marker 46 is programmed with the geo location and/or the user-friendly name of the location and the location marker 46 is affixed to the building or site in that location. The information can include the type of asset, e.g., smoke detector, exit sign, an id of the asset, the geo coordinates, location in the premises. This process is repeated in each location around the site or building so that the building or site has adequate coverage to allow tracking during the tracking phase of operation.

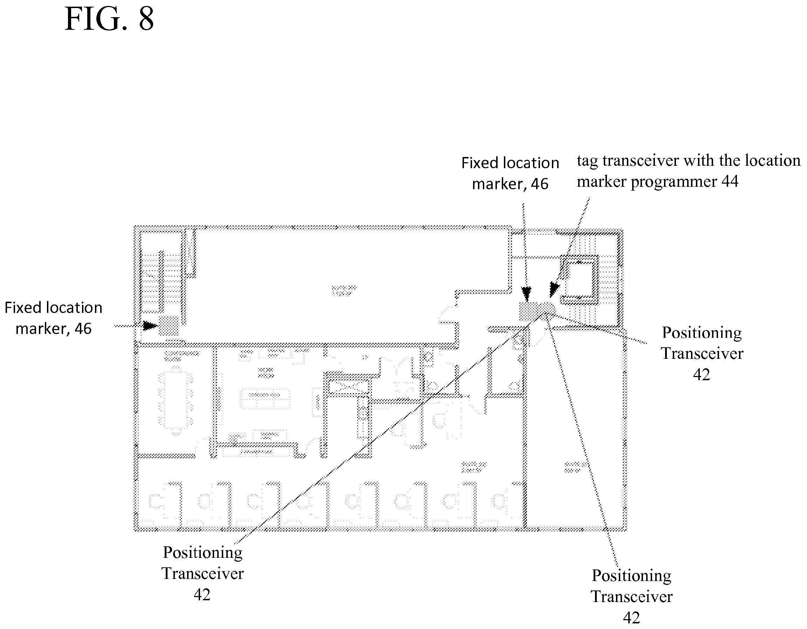

Referring now to FIG. 8 a typical example of the programming of location markers 46 during the deployment phase of operation is shown. In this case there are 3 Positioning Transceivers 42 that are placed in 3 locations outside of the building. Two location markers 46 are shown with both geo coordinates and user-friendly location names. The location marker 46 (right side of the diagram) is being programmed by the tag transceiver with the location marker programmer 44.

According to a non-claimed example of the present disclosure, in the event that it is impractical or otherwise undesirable to determine the geo coordinates of the various location markers 46 around the site or building, the tags can be programmed with just the user-friendly location information. Even without the geo coordinates, the friendly location name provides significant value for tracking the location during the tracking phase of operation.

### The Tracking Phase of Operation:

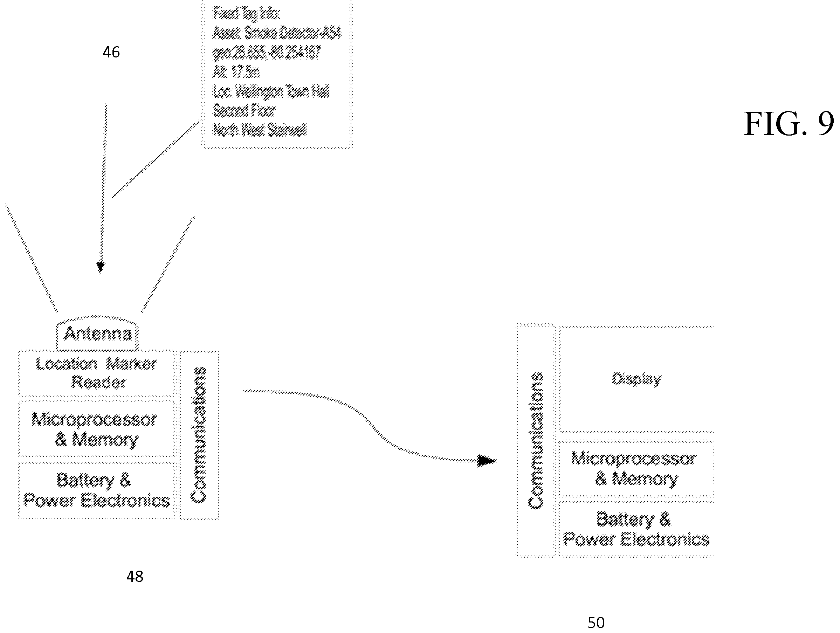

Referring now to FIG. 9, during the tracking phase of operation, the mobile tracking system 48 is deployed into the site usually affixed to a person or high value asset that is being tracked. As the mobile tracking system 48 moves around the site, the mobile tracking system 48 moves near various location markers 46 that are located around the building or site. As the mobile tracking system 48 reads the various location markers, the mobile tracking system 48 produces a record of the time that the location marker 46 was read and the geo coordinate information and the user-friendly location name on each of the location markers 46. This information is communicated over a communication channel to the mobile monitoring and display system 50. In some cases, more than one mobile tracking system. 48 may be deployed to a site or building that may be individually displayed on the Mobile Monitoring and Display System 50.

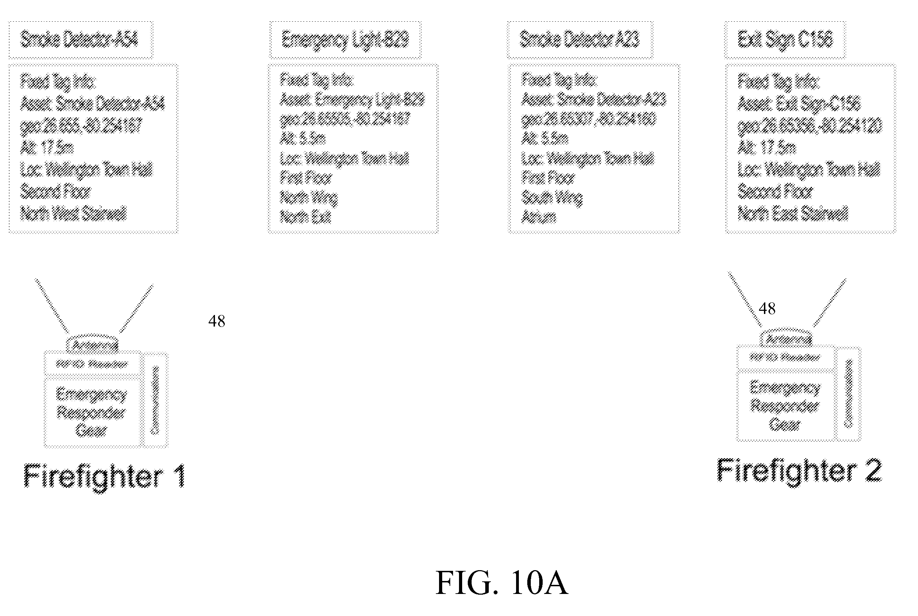

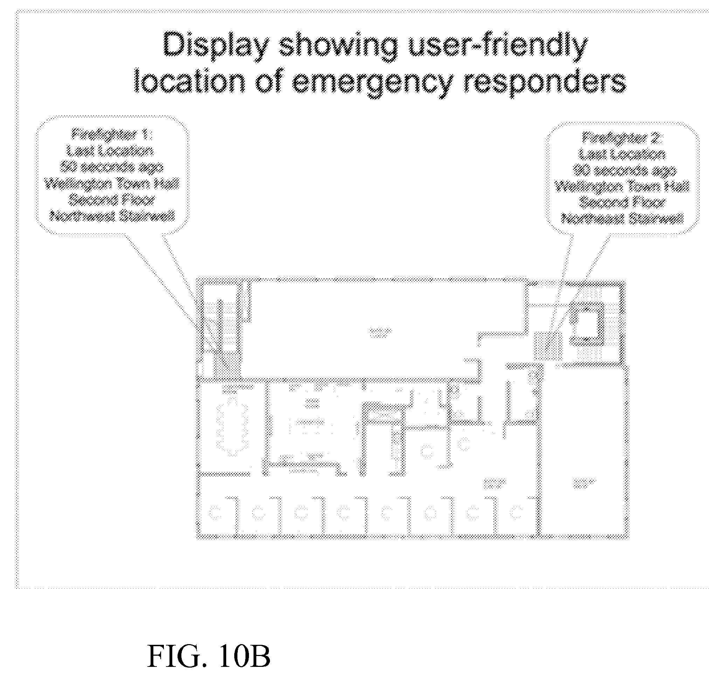

Referring now to FIGS. 10A and 10B, a potential use case with emergency personnel carrying a mobile tracking system 48 as part of their gear is shown. In this example, there are 2 firefighters that are located in two different locations around the building. In this example, the mobile monitoring and display system 50 (FIG. 10B) displays just the friendly name of the last known location of the firefighters and how long ago they were at that location.

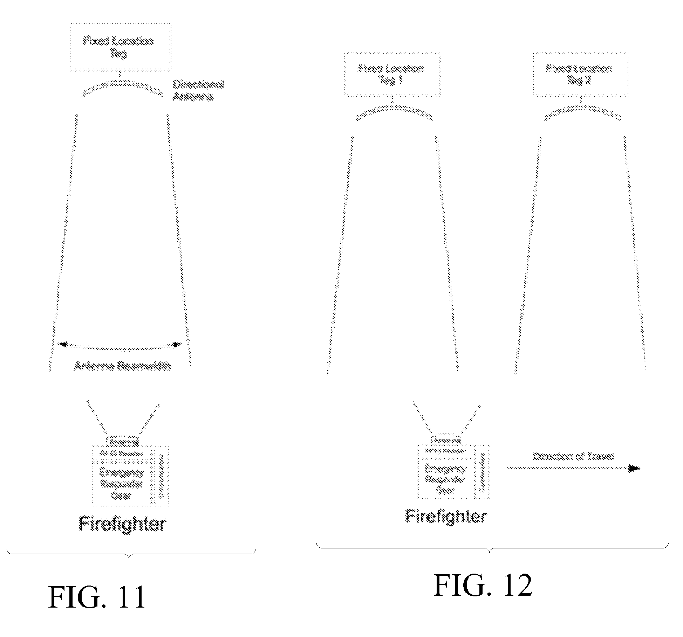

The mobile tracking system 48 uses location markers (not shown) that may have significant over range of operation allowing the location markers to be read from a large distance away, which could pose a problem. There are several approaches to manage that over-range: The power in the location marker reader may be adjusted down to read only a short distance. The antenna size in the location marker and/or the location marker reader may be configured to minimize over-range. The location marker may be configured with a narrow beam antenna that could be directed at a specific region to narrow the read-zone and reduce the over-range as shown in FIG. 11. Also as shown in FIG. 12, two location markers 46 with narrow beam antennas may be placed nearby one another which could provide direction of motion and the speed of the mobile tracking system 48 as it passes within range of both location markers 46. The location marker 46 can contain accuracy information that allows the zone of presence around the location marker 46 to be displayed along with the location information. The location marker 46 may store information using encryption techniques that allow only authorized users to read the information.

The location tracking system 40 may be implemented using any appropriate type of computing device, such as a mainframe work station, a personal computer, a server, a portable computing device, or any other type of intelligent device capable of executing instructions, connecting to a network, and forwarding data packets through the network and can execute any appropriate computer programs to generate, receive, and transmit data packets for use on the network.

Each of processes discussed above may be stored on one or more non-transitory machine-readable media, such as computer memory persistent or non-persistent to store executable instructions. Each of these devices may also include one or more processing devices (e.g., microprocessors, programmable logic, application-specific integrated circuits, and so forth) for executing the instructions to perform all or part of the functions described herein.

Elements of different implementations described herein may be combined to form other embodiments not specifically set forth above. Elements may be left out of the structures described herein without adversely affecting their operation. Furthermore, various separate elements may be combined into one or more individual elements to perform the functions described herein.

### Example application

An example, non-limiting application of the WSN is in a security system for intrusion detection, fire, toxic gas, monitor, etc. installed at one or more premises such as one or more residential houses or building(s) and especially in, e.g., commercial, industrial, buildings, complexes, etc.

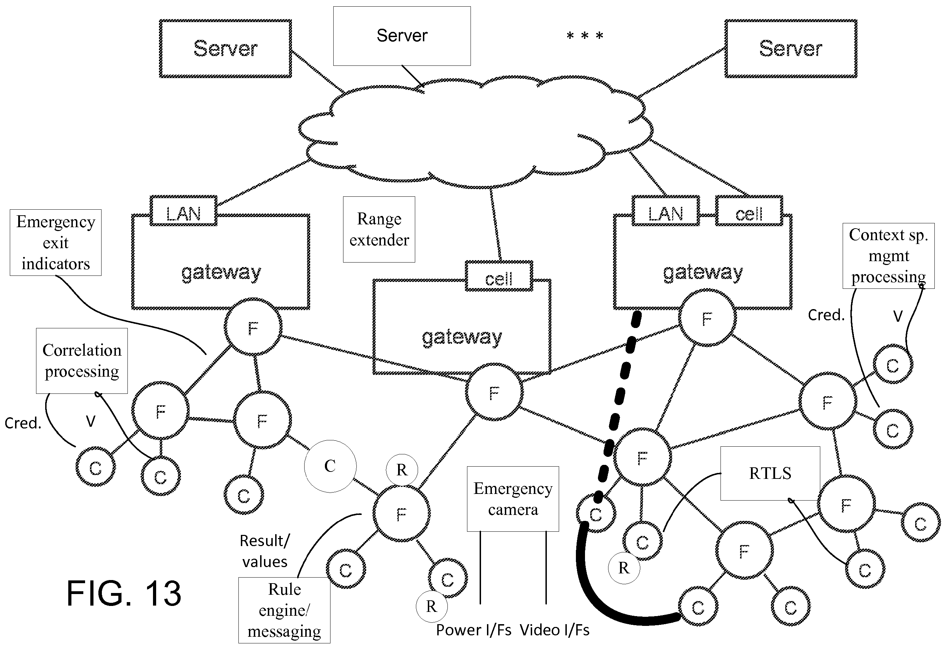

FIG. 13 shows an example of a security system having features of the WSN described with respect to FIG. 1 and having the various functionalities described herein. As shown in FIG. 13, correlation processing receives inputs from certain constrained nodes (although these can also be fully functional nodes). These inputs may include credential information and video information, and the correlation processing may produce correlated results that are sent over the network. Context management processing receives inputs from certain constrained nodes (although these can also be fully functional nodes) e.g., credential information and video and grouping information, and performs context processing with results sent over the network. The network supports operation of emergency exit indicators; emergency cameras as well as distributed rule processing and rule engine/messaging processing. Range extenders are used with e.g., gateways, and a real time location system receives inputs from various sensors (e.g., constrained type) as shown. Servers interface to the WSN via a cloud computing configuration and parts of some networks can be run as sub-nets.

The sensors provide in addition to an indication that something is detected in an area within the range of the sensors, detailed additional information that can be used to evaluate what that indication may be without the intrusion detection panel being required to perform extensive analysis of inputs to the particular sensor.

For example, a motion detector could be configured to analyze the heat signature of a warm body moving in a room to determine if the body is that of a human or a pet. Results of that analysis would be a message or data that conveys information about the body detected. Various sensors thus are used to sense sound, motion, vibration, pressure, heat, images, and so forth, in an appropriate combination to detect a true or verified alarm condition at the intrusion detection panel.

Recognition software can be used to discriminate between objects that are a human and objects that are an animal; further facial recognition software can be built into video cameras and used to verify that the perimeter intrusion was the result of a recognized, authorized individual. Such video cameras would comprise a processor and memory and the recognition software to process inputs (captured images) by the camera and produce the metadata to convey information regarding recognition or lack of recognition of an individual captured by the video camera. The processing could also alternatively or in addition include information regarding characteristic of the individual in the area captured/monitored by the video camera. Thus, depending on the circumstances, the information would be either metadata received from enhanced motion detectors and video cameras that performed enhanced analysis on inputs to the sensor that gives characteristics of the perimeter intrusion or a metadata resulting from very complex processing that seeks to establish recognition of the object.

Sensor devices can integrate multiple sensors to generate more complex outputs so that the intrusion detection panel can utilize its processing capabilities to execute algorithms that analyze the environment by building virtual images or signatures of the environment to make an intelligent decision about the validity of a breach.

Memory stores program instructions and data used by the processor of the intrusion detection panel. The memory may be a suitable combination of random access memory and read-only memory, and may host suitable program instructions (e.g. firmware or operating software), and configuration and operating data and may be organized as a file system or otherwise. The stored program instruction may include one or more authentication processes for authenticating one or more users. The program instructions stored in the memory of the panel may further store software components allowing network communications and establishment of connections to the data network. The software components may, for example, include an internet protocol (IP) stack, as well as driver components for the various interfaces, including the interfaces and the keypad . Other software components suitable for establishing a connection and communicating across network will be apparent to those of ordinary skill.

Program instructions stored in the memory, along with configuration data may control overall operation of the panel.

The monitoring server includes one or more processing devices (e.g., microprocessors), a network interface and a memory (all not illustrated). The monitoring server may physically take the form of a rack mounted card and may be in communication with one or more operator terminals (not shown). An example monitoring server is a SURGARD^{™} SG-System III Virtual, or similar system.

The processor of each monitoring server acts as a controller for each monitoring server, and is in communication with, and controls overall operation, of each server. The processor may include, or be in communication with, the memory that stores processor executable instructions controlling the overall operation of the monitoring server. Suitable software enable each monitoring server to receive alarms and cause appropriate actions to occur. Software may include a suitable Internet protocol (IP) stack and applications/clients.

Each monitoring server of the central monitoring station may be associated with an IP address and port(s) by which it communicates with the control panels and/or the user devices to handle alarm events, etc. The monitoring server address may be static, and thus always identify a particular one of monitoring server to the intrusion detection panels. Alternatively, dynamic addresses could be used, and associated with static domain names, resolved through a domain name service.

The network interface card interfaces with the network to receive incoming signals, and may for example take the form of an Ethernet network interface card (NIC). The servers may be computers, thin-clients, or the like, to which received data representative of an alarm event is passed for handling by human operators. The monitoring station may further include, or have access to, a subscriber database that includes a database under control of a database engine. The database may contain entries corresponding to the various subscriber devices/processes to panels like the panel that are serviced by the monitoring station.

All or part of the processes described herein and their various modifications (hereinafter referred to as "the processes") can be implemented, at least in part, via a computer program product, i.e., a computer program tangibly embodied in one or more tangible, physical hardware storage devices that are computer and/or machine-readable storage devices for execution by, or to control the operation of, data processing apparatus, e.g., a programmable processor, a computer, or multiple computers. A computer program can be written in any form of programming language, including compiled or interpreted languages, and it can be deployed in any form, including as a stand-alone program or as a module, component, subroutine, or other unit suitable for use in a computing environment. A computer program can be deployed to be executed on one computer or on multiple computers at one site or distributed across multiple sites and interconnected by a network.

Actions associated with implementing the processes can be performed by one or more programmable processors executing one or more computer programs to perform the functions of the calibration process. All or part of the processes can be implemented as, special purpose logic circuitry, e.g., an FPGA (field programmable gate array) and/or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only storage area or a random access storage area or both. Elements of a computer (including a server) include one or more processors for executing instructions and one or more storage area devices for storing instructions and data. Generally, a computer will also include, or be operatively coupled to receive data from, or transfer data to, or both, one or more machine-readable storage media, such as mass storage devices for storing data, e.g., magnetic, magneto-optical disks, or optical disks.

Tangible, physical hardware storage devices that are suitable for embodying computer program instructions and data include all forms of non-volatile storage, including by way of example, semiconductor storage area devices, e.g., EPROM, EEPROM, and flash storage area devices; magnetic disks, e.g., internal hard disks or removable disks; magneto-optical disks; and CD-ROM and DVD-ROM disks and volatile computer memory, e.g., RAM such as static and dynamic RAM, as well as erasable memory, e.g., flash memory.

## Claims

1. A real-time location system (40) comprises:
- a plurality of positioning transceiver systems (42) configured to determine, for each positioning transceiver system (42), its corresponding position, relative to a global coordinate system, during set up of the real-time location system (40);
- a tag transceiver with location marker programmer (44) configured to determine the location of the tag transceiver relative to global position coordinates and configured to:
- provide global coordinate location information for programming a plurality of location markers (46) with location information;
- receive user-friendly location information that is a user supplied name of the location; and
- program the plurality of location markers (46) with location information;
- the plurality of location markers (46) programmed by the tag transceiver with location marker programmer (44), the plurality of location markers (46) being programmed with location information of the locations that the location markers (46) are affixed to around a site, and the user-friendly location information;
- a mobile tracking system (48) configured to read location information from nearby location markers (46) that are placed around the site; and
- a mobile monitoring and display system (50) configured to receive signals from the mobile tracking system (48) to display status and geo coordinates of the mobile tracking system (48).

2. The real-time location system (40) of claim 1,
wherein the location markers (46) are read by the mobile tracking system (48), and the mobile tracking system (48) produces for a location marker (46), a record of a time that the location marker (46) was read and the geo coordinate information and/or the user-friendly location name on the location marker (46).

3. The real-time location system (40) of claim 1,
wherein the positioning transceiver systems are placed throughout the site.

4. The real-time location system (40) of claim 1,
wherein the location information is geo coordinate information and/or user-friendly location information.

5. The real-time location system (40) of claim 1,
wherein the location markers (46) comprises:
- circuitry including a processor and memory that controls location marker (46) and is configured to be programmed with geo coordinate information and/or the user-friendly location information, and an antenna for the communication of location information;
- an antenna; and
- communication circuitry coupled to the antenna and the circuitry including the processor for communication of the global coordinate location information and/or the user-friendly location information.

6. The real-time location system (40) of claim 1,
wherein the mobile tracking system (48) further comprises:
- a location marker reader that reads information from location markers (46) during tracking phase of operation;
- an antenna that facilitates communication with the location markers (46);
- circuitry including a microprocessor and memory that controls the operation of the mobile tracking system (48); and
- a communications block adapted to provide a communication channel between the mobile tracking system (48) and the mobile monitoring and display system (50).

7. The real-time location system (40) of claim 1,
wherein each positioning transceiver system (42) further comprises:
- a positioning element arranged to determine a distance or angle from the positioning element to a traditional tag that is used with a traditional real-time location tracking system (40) that has position information for each of the locating elements, said information being fixed at known locations or determined at a setup time;
- a global positioning transceiver; and
- circuitry including a microprocessor and memory subsystem to control the positioning transceiver system (42) and for communication of data to a tag transceiver with location marker programmer (44).

8. The real-time location system (40) of claim 1,
wherein the tag transceiver with location marker programmer (44) further comprises:
- a tag element to determine the location of the tag transceiver with location marker programmer (44), relative to the global position coordinates, and a location marker programmer to program location markers (46) with the global positioning coordinates and with the location marker programmer further configured to program the location markers (46) with user-friendly location information.

9. The real-time location system (40) of one of claims 1 to 8,
wherein the mobile monitoring and display system (50) comprises a microprocessor and memory which control the functionality of the system to display status and geo coordinates of the mobile tracking system (48).

10. A method of real-time location tracking using the real-time location system (40) of claim 1, the method comprising:
- deploying the plurality of positioning transceiver systems (42) configured to determine for each positioning transceiver system (42), its corresponding position, relative to a global coordinate system, during set up of the real-time location tracking;
- determining by the tag transceiver with the location marker programmer (44), location of the tag transceiver relative to the global position coordinate system from the plurality of positioning transceiver systems (42), receiving user-friendly location information that is a user supplied name of the location and to program the plurality of location markers (46) with location information;
- deploying the plurality of location markers (46) that are programmed by the tag transceiver with the location marker programmer (44) with location information of the locations that the location markers (46) are affixed to around a site;
- deploying the mobile tracking system (48) to read location information from nearby location markers (46) that are placed around the site; and
- receiving by the mobile monitoring and display system (50) signals from the mobile tracking system (48) to display status and geo coordinates of the mobile tracking system (48).

11. The method of claim 10, further comprising:
- reading the location markers (46) by the mobile tracking system (48); and
- storing by the mobile tracking system (48) for a location marker (46), a record of the time that the location marker (46) was read and the geo coordinate information and/or the user-friendly location information.

## Patentansprüche

1. Echtzeit-Standortsystem (40), umfassend:
- eine Vielzahl von Positionssendeempfängersystemen (42), die ausgelegt sind, um während der Einrichtung des Echtzeit-Standortsystems (40) für jedes Positionssendeempfängersystem (42) die entsprechende Position in Bezug zu einem globalen Koordinatensystem zu bestimmen;
- einen Tag-Sendeempfänger mit einem Lokalisierungsmarkerprogrammierer (44), der ausgelegt ist, um den Standort des Tag-Sendeempfängers in Bezug zu globalen Positionskoordinaten zu bestimmen, und der ausgelegt ist, um:
- globale Koordinatenpositionsinformationen zur Programmierung einer Vielzahl von Standortmarkern (46) mit Standortinformationen bereitzustellen;
- benutzerfreundliche Standortinformationen zu empfangen, bei denen es sich um einen vom Benutzer bereitgestellten Namen des Standorts handelt; und
- die Vielzahl von Standortmarkern (46) mit Standortinformationen zu programmieren;
- die Vielzahl von Standortmarkern (46), die durch den Tag-Sendeempfänger mit einem Standortmarkerprogrammierer (44) programmiert sind, wobei die Vielzahl von Standortmarkern (46) mit Standortinformationen der Standorte, an denen die Standortmarker (46) um eine Stelle herum angebracht sind, und den benutzerfreundlichen Standortinformationen programmiert ist;
- ein mobiles Trackingsystem (48), das ausgelegt ist, um Standortinformationen von nahegelegenen Standortmarkern (46) auszulesen, die um die Stelle herum platziert sind; und
- ein mobiles Überwachungs- und Anzeigesystem (50), das ausgelegt ist, um Signale vom mobilen Trackingsystem (48) zu empfangen, um den Status und die Geokoordinaten des mobilen Trackingsystems (48) anzuzeigen.

2. Echtzeit-Standortsystem (40) nach Anspruch 1,
wobei die Standortmarker (46) vom mobilen Trackingsystem (48) ausgelesen werden und das mobile Trackingsystem (48) für einen Standortmarker (46) ein Protokoll einer Zeit, zu welcher der Standortmarker (46) ausgelesen wurde, und die Geokoordinateninformationen und/oder den benutzerfreundlichen Standortnamen am Standortmarker (46) erzeugt.

3. Echtzeit-Standortsystem (40) nach Anspruch 1,
wobei die Positionssendeempfängersysteme überall an der Stelle platziert sind.

4. Echtzeit-Standortsystem (40) nach Anspruch 1,
wobei die Standortinformationen Geokoordinaten und/oder benutzerfreundliche Standortinformationen sind.

5. Echtzeit-Standortsystem (40) nach Anspruch 1,
wobei der Standortmarker (46) umfasst:
- eine Schaltung, umfassend einen Prozessor und einen Speicher, die den Standortmarker (46) steuern und ausgelegt sind, um mit Geokoordinateninformationen und/oder den benutzerfreundlichen Standortinformationen programmiert zu werden, und eine Antenne für die Übertragung der Standortinformationen;
- eine Antenne; und
- eine Übertragungsschaltung, die mit der Antenne und der Schaltung, die den Prozessor für die Übertragung der globalen Koordinatenstandortinformationen und/oder der benutzerfreundlichen Standortinformationen umfasst, gekoppelt ist.

6. Echtzeit-Standortsystem (40) nach Anspruch 1,
wobei das mobile Trackingsystem (48) außerdem umfasst:
- einen Standortmarkerleser, der Informationen aus Standortmarkern (46) während der Trackingphase des Betriebs ausliest;
- eine Antenne, welche die Übertragung der Standortmarker (46) ermöglicht;
- eine Schaltung, umfassend einen Mikroprozessor und einen Speicher, die den Betrieb des mobilen Trackingsystems (48) steuern; und
- einen Übertragungsblock, der angepasst ist, um einen Übertragungskanal zwischen dem mobilen Trackingsystem (48) und dem mobilen Überwachungs- und Anzeigesystem (50) bereitzustellen.

7. Echtzeit-Standortsystem (40) nach Anspruch 1,
wobei jedes Positionssendeempfängersystem (42) außerdem umfasst:
- ein Positionselement, das angeordnet ist, um den Abstand oder Winkel des Positionselements zu einem herkömmlichen Tag zu bestimmen, das mit einem herkömmlichen Echtzeit-Standorttrackingsystem (40) verwendet wird, das Positionsinformationen für jedes der Lokalisierungselemente hat, wobei die Informationen an bekannten Standorten festgelegt sind oder zum Zeitpunkt der Einrichtung bestimmt werden;
- einen globalen Positionssendeempfänger; und
- eine Schaltung, die ein Mikroprozessor- und Speichersubsystem umfasst, um das Positionssendeempfängersystem (42) zu steuern und um Daten an einen Tag-Sendeempfänger mit einem Standortmarkerprogrammierer (44) zu übertragen.

8. Echtzeit-Standortsystem (40) nach Anspruch 1,
wobei der Tag-Sendeempfänger mit einem Standortmarkerprogrammierer (44) außerdem umfasst:
- ein Tag-Element, um den Standort des Tag-Sendeempfängers mit einem Standortmarkerprogrammierer (44) in Bezug auf die globalen Positionskoordinaten zu bestimmen, und einen Standortmarkerporgrammierer, um Standortmarker (46) mit den globalen Positionskoordinaten zu programmieren, wobei der Standortmarkerprogrammierer außerdem ausgelegt ist, um die Standortmarker (46) mit benutzerfreundlichen Standortinformationen zu programmieren.

9. Echtzeit-Standortsystem (40) nach einem der Ansprüche 1 bis 8,
wobei das mobile Überwachungs- und Anzeigesystem (50) einen Mikroprozessor und einen Speicher umfasst, welche die Funktionalität des Systems steuern, den Status und die Geokoordinaten des mobilen Trackingsystems (48) anzuzeigen.

10. Verfahren zum Echtzeit-Standorttracking unter Verwendung eines Echtzeit-Standortsystems (40) nach Anspruch 1, wobei das Verfahren umfasst:
- Auslösen der Vielzahl von Positionssendeempfängersystemen (42), die ausgelegt sind, um während der Einrichtung des Echtzeit-Standorttrackings für jedes Positionssendeempfängersystem (42) die entsprechende Position in Bezug zu einem globalen Koordinatensystem zu bestimmen;
- Bestimmen, mittels des Tag-Sendeempfängers mit dem Lokalisierungsmarkerprogrammierer (44), des Standorts des Tag-Sendeempfängers in Bezug zum globalen Positionskoordinatensystem aus der Vielzahl von Positionssendeempfängersystemen (42), wobei benutzerfreundliche Standortinformationen empfangen werden, bei denen es sich um einen vom Benutzer bereitgestellten Namen des Standorts handelt, und die Vielzahl von Standortmarkern (46) mit Standortinformationen programmiert wird,
- Auslösen der Vielzahl von Standortmarkern (46), die vom Tag-Sendeempfänger mit einem Standortmarkerprogrammierer (44) mit Standortinformationen der Standorte programmiert werden, an denen die Standortmarker (46) um eine Stelle herum angebracht sind;
- Auslösen des mobilen Trackingsystems (48), um Standortinformationen von nahegelegenen Standortmarkern (46) auszulesen, die um die Stelle herum platziert sind; und
- Empfangen, mittels des mobilen Überwachungs- und Anzeigesystem (50), von Signalen vom mobilen Trackingsystem (48), um den Status und die Geokoordinaten des mobilen Trackingsystems (48) anzuzeigen.

11. Verfahren nach Anspruch 10, außerdem umfassend:
- Auslesen der Standortmarker (46) durch das mobile Trackingsystem (48); und
- Speichern, für einen Standortmarker (46), durch das mobile Trackingsystem (48), eines Protokolls der Zeit, zu welcher der Standortmarker (46) ausgelesen wurde, und der Geokoordinateninformationen und/oder der benutzerfreundlichen Standortinformationen.

## Revendications

1. Système de localisation en temps réel (40) comprenant :
- une pluralité de systèmes émetteurs-récepteurs de positionnement (42) configurés pour déterminer, pour chaque système émetteur-récepteur de positionnement (42), sa position correspondante, par rapport à un système de coordonnées global, pendant la configuration du système de localisation en temps réel (40) ;
- un émetteur-récepteur d'étiquettes avec programmeur de marqueurs de localisation (44) configuré pour déterminer la localisation de l'émetteur-récepteur d'étiquettes par rapport aux coordonnées de position globale et configuré pour :
- fournir des informations de localisation par coordonnées globales permettant de programmer une pluralité de marqueurs de localisation (46) avec des informations de localisation ;
- recevoir des informations de localisation faciles à utiliser qui sont un nom de localisation fourni par l'utilisateur ; et
- programmer la pluralité de marqueurs de localisation (46) avec des informations de localisation ;
- la pluralité de marqueurs de localisation (46) programmés par l'émetteur-récepteur d'étiquettes avec programmeur de marqueurs de localisation (44), la pluralité de marqueurs de localisation (46) étant programmée avec les informations de localisation des localisations sur lesquelles les marqueurs de localisation (46) sont apposés autour d'un site, et les informations de localisation faciles à utiliser ;
- un système de suivi mobile (48) configuré pour lire les informations de localisation à partir des marqueurs de localisation (46) à proximité qui sont placés autour du site ; et
- un système de surveillance et d'affichage mobile (50) configuré pour recevoir des signaux provenant du système de suivi mobile (48) permettant d'afficher l'état et les coordonnées géographiques du système de suivi mobile (48).

2. Système de localisation en temps réel (40) selon la revendication 1,
dans lequel les marqueurs de localisation (46) sont lus par le système de suivi mobile (48), et le système de suivi mobile (48) produit pour un marqueur de localisation (46), un enregistrement d'une heure à laquelle le marqueur de localisation (46) a été lu et les informations de coordonnées géographiques et/ou le nom de localisation facile à utiliser sur le marqueur de localisation (46).

3. Système de localisation en temps réel (40) selon la revendication 1,
dans lequel les systèmes d'émetteurs-récepteurs de positionnement sont placés sur l'ensemble du site.

4. Système de localisation en temps réel (40) selon la revendication 1,
dans lequel les informations de localisation sont des informations de coordonnées géographiques et/ou des informations de localisation faciles à utiliser.

5. Système de localisation en temps réel (40) selon la revendication 1,
dans lequel les marqueurs de localisation (46) comprennent :
- un ensemble de circuits comportant un processeur et une mémoire qui contrôle le marqueur de localisation (46) et qui est configuré pour être programmé avec des informations de coordonnées géographiques et/ou des informations de localisation faciles à utiliser, et une antenne pour la communication d'informations de localisation ;
- une antenne ; et
- un ensemble de circuits de communication couplé à l'antenne et à l'ensemble de circuits comportant le processeur pour la communication des informations de localisation par coordonnées globales et/ou des informations de localisation faciles à utiliser.

6. Système de localisation en temps réel (40) selon la revendication 1,
dans lequel le système de suivi mobile (48) comprend en outre :
- un lecteur de marqueurs de localisation qui lit les informations des marqueurs de localisation (46) pendant la phase de suivi de l'opération ;
- une antenne qui facilite la communication avec les marqueurs de localisation (46) ;
- un ensemble de circuits comportant un microprocesseur et une mémoire qui contrôle le fonctionnement du système de suivi mobile (48) ; et
- un bloc de communications adapté pour fournir un canal de communication entre le système de suivi mobile (48) et le système de surveillance et d'affichage mobile (50).

7. Système de localisation en temps réel (40) selon la revendication 1,
dans lequel chaque système émetteur-récepteur de positionnement (42) comprend en outre :
- un élément de positionnement disposé pour déterminer une distance ou un angle entre l'élément de positionnement et une étiquette traditionnelle qui est utilisée avec un système de localisation en temps réel traditionnel (40) qui a des informations sur la position de chacun des éléments de localisation, lesdites informations étant fixées à des localisations connues ou déterminées à une heure de configuration ;
- un émetteur-récepteur de positionnement global ; et
- un ensemble de circuits comportant un microprocesseur et un sous-système de mémoire pour contrôler le système émetteur-récepteur de positionnement (42) et pour communiquer des données à un émetteur-récepteur d'étiquettes avec programmeur de marqueurs de localisation (44).

8. Système de localisation en temps réel (40) selon la revendication 1,
dans lequel l'émetteur-récepteur avec programmeur de marqueurs de localisation (44) comprend en outre :
- un élément d'étiquette pour déterminer la localisation de l'émetteur-récepteur d'étiquettes avec programmeur de marqueurs de localisation (44), par rapport aux coordonnées de positionnement global, et un programmeur de marqueurs de localisation pour programmer les marqueurs de localisation (46) avec les coordonnées de positionnement global et avec le programmeur de marqueurs de localisation configuré en outre pour programmer les marqueurs de localisation (46) avec des informations de localisation faciles à utiliser.

9. Système de localisation en temps réel (40) selon l'une des revendications 1 à 8,
dans lequel le système de surveillance et d'affichage mobile (50) comprend un microprocesseur et une mémoire qui contrôlent la fonctionnalité du système permettant d'afficher l'état et les coordonnées géographiques du système de suivi mobile (48).

10. Procédé de suivi de localisation en temps réel utilisant le système de localisation en temps réel (40) selon la revendication 1, le procédé comprenant :
- le déploiement de la pluralité de systèmes émetteurs-récepteurs de positionnement (42) configurés pour déterminer, pour chaque système émetteur-récepteur de positionnement (42), sa position correspondante, par rapport à un système de coordonnées global, pendant la configuration du suivi de localisation en temps réel ;
- la détermination par l'émetteur-récepteur d'étiquettes avec le programmeur de marqueurs de localisation (44), de la localisation de l'émetteur-récepteur d'étiquettes par rapport au système de coordonnées de position globale de la pluralité de systèmes émetteurs-récepteurs de positionnement (42), la réception d'informations de localisation faciles à utiliser, qui sont un nom de localisation fourni par l'utilisateur, et la programmation de la pluralité de marqueurs de localisation (46) avec des informations de localisation ;
- le déploiement de la pluralité de marqueurs de localisation (46) qui sont programmés par l'émetteur-récepteur d'étiquettes avec le programmeur de marqueurs de localisation (44) avec les informations de localisation des localisations sur lesquelles les marqueurs de localisation (46) sont apposés autour d'un site ;
- le déploiement du système de suivi mobile (48) pour lire les informations de localisation à partir des marqueurs de localisation (46) à proximité qui sont placés autour du site ; et
- la réception par le système de surveillance et d'affichage mobile (50) de signaux provenant du système de suivi mobile (48) permettant d'afficher l'état et les coordonnées géographiques du système de suivi mobile (48).

11. Procédé selon la revendication 10, comprenant en outre :
- la lecture des marqueurs de localisation (46) par le système de suivi mobile (48) ; et
- le stockage par le système de suivi mobile (48) pour un marqueur de localisation (46), d'un enregistrement de l'heure à laquelle le marqueur de localisation (46) a été lu et des informations de coordonnées géographiques et/ou des informations de localisation faciles à utiliser.
